Europäisches Patentamt

European Patent Office

Office européen des brevets

(11) Publication number: **0 130 064**
**B1**

# EUROPEAN PATENT SPECIFICATION

(45) Date of publication of patent specification: 31.08.88

(51) Int. Cl.⁴: **C 11 D 3/386, C 12 N 9/20**

(21) Application number: **84304236.7**

(22) Date of filing: **22.06.84**

(54) Improvements in and relating to an enzymatic detergent additive, a detergent, and a washing method.

(30) Priority: **23.06.83 DK 2890/83**

(43) Date of publication of application:
**02.01.85 Bulletin 85/01**

(45) Publication of the grant of the patent:
**31.08.88 Bulletin 88/35**

(84) Designated Contracting States:
**AT BE CH DE FR GB IT LI NL**

(56) References cited:
**DE-A-2 042 650**
**FR-A-2 097 842**
**GB-A-1 401 312**

**Arch. of Biochem; 26 (1950) p. 382-400**
**Indian Journal of Experimental Biology, Vol. 11**
**(1973) p. 37-39; Agric. Biol.-Chem., 43(1) 1979,**
**p. 2126, Table I.**

(73) Proprietor: **NOVO INDUSTRI A/S**
**Novo Allé**
**DK-2880 Bagsvaerd (DK)**

(72) Inventor: **Nielsen, Ruby Ione**
**Gedebakken 9**
**DK-3520 Farum (DK)**

(74) Representative: **Brown, John David et al**
**FORRESTER & BOEHMERT Widenmayerstrasse**
**4/I**
**D-8000 München 22 (DE)**

Courier Press, Leamington Spa, England.

## Description

The field of enzymatic additives has been rapidly growing during the last decades. Reference is made to e.g. the article "How Enzymes Got into Detergents", Vol. 12, Developments in Industrial Microbiology, a publication of the Society for Industrial Microbiology, American Institute of Biological Sciences, Washington, D.C. 1971, by Claus Dambmann, Poul Holm, Villy Jensen, and Mogens Hilmer Nielsen.

The most common enzymatic detergent additive is a proteolytic additive, but also lipolytic detergent additives are described, e.g. in U.S. Patent No. 4,011,116, column 4, line 65 to column 5, line 68, and British Patent No. 1,293,613, page 2, lines 6 to 29.

Also, a comprehensive review article of lipases as detergent additives written by Hans Andree et al. is to be found in the Journal of Applied Biochemistry, 2, 218—229 (1980), entitled "Lipases as Detergent Components".

If the washing process is conducted at high temperature and high alkalinity, the fat containing dirt will be dissolved by saponification. However, due to the energy crisis, low temperature washing processes (around 60°C and below) are generally preferred, and at these low temperatures the known lipases are able to dissolve only a part of the fat containing dirt.

The efficiency of lipolytic enzymatic detergent additives can conveniently be measured by means of EMPA (Eidgenössische Materialprüfungsund Versuchsanstalt, St. Gallen, Switzerland) swatches Nos. 101 (olive oil/cotton) and 102 (olive oil/wool) by adaptation of the procedure described in British Patent No. 1,361,386 (especially pages 4 and 7) and US patent No. 3,723,250 (especially col. 15—19). In this way it appears that the enzymes known as lipolytic detergent additives are rather unsatisfactory in the sense that they exhibit an unsatisfactorily low lipolytic cleaning efficiency, as reflected in the low value of the differential remittance value $\Delta R$ at economically reasonable lipase activities in the washing solution.

Thus, a need exists for a lipolytic detergent additive which exhibits a considerably better lipolytic cleaning efficiency, corresponding to a considerably higher differential remittance value, $\Delta R$, at economically reasonable lipase activities in the washing solution.

Now, according to the first aspect of the invention, a lipolytic detergent additive has been found which exhibits a considerably better lipolytic cleaning efficiency, corresponding to a considerably higher differential remittance value, $\Delta R$, at economically reasonable lipase activities in the washing solution—this lipolytic detergent additive being characterized by the fact that the lipase is producible by means of a lipase producing strain of *Fusarium oxysporum*.

The definition of the species *Fusarium oxysporum* has changed somewhat during the last decades. However, for the purposes of this invention, the definition of the species *Fusarium oxysporum* is the definition set forth in "The Genus *Fusarium*", C. Booth, CMI, 1971.

In relation to certain strains of *Fusarium oxysporum* a lipase formation is described, *vide* e.g. Agric. Biol. Chem. 43 (10) (1979), 2126, Table I where a *Fusarium lini* lipase is indicated (according to the above definition of *Fusarium oxysporum*, *Fusarium lini* now belongs to *Fusarium oxysporum*), and Indian Journal of Experimental Biology, Vol. 11 (1973), p. 37—39 with the title "Lipids and Lipase Activity in Strains of *Fusarium vasinfectum*" (according to the above definition of *Fusarium oxysporum*, *Fusarium vasinfectum* now belongs to *Fusarium oxysporum*).

Some strains belonging to *Fusarium oxysporum* are poor producers of lipase. The lipase producing strains of *Fusarium oxysporum* for the purposes of this invention produce more than 10 LU/ml (the LU being the Lipase Units defined later in this specification) under the following standard fermentation conditions:

A substrate intended for shaking flasks is prepared with the following ingredients in grams per litre:

| Soy bean meal | 45 |
|---|---|
| Glucose | 70 |
| $KH_2PO_4$ | 2 |
| $Na_2HPO_4$ | 3 |
| Soy oil | 5 |

Sterilization took place at 121°C for 40 minutes. A 500 ml Erlenmeyer flask with 100 ml of substrate was inoculated with spores from an agar slant previously inoculated with the strain of *Fusarium oxysporum* to be tested for lipase production. The flasks were shaken at 230 rpm and at 30°C for 5 days whereafter the lipase yield was determined. Reference is made to example 29.

Thus, it has surprisingly been found that the detergent additive according to the invention exhibits a drastically improved lipolytic cleaning efficiency which will appear from documentation presented later in this specification.

With regard to the *Fusarium oxysporum* strains DSM 2672, ATCC 7808, CBS 620.72, CBS 645.78, and CBS 794.70, it has been found that the average value of the pH activity optimum is around 9—11, and that the average temperature activity optimum is around 35—50°C (time of analysis: 20 minutes). On the basis of crossed immunoelectrophoresis with antibodies produced from the lipase from DSM 2672 it appears that the lipases originating from the above indicated five strains are identical or partially identical. On the basis of the above findings the conclusion may be drawn that the active component of the enzymatic detergent additive according to the invention is a group of closely related lipases.

The lipase activity is determined according to the NOVO method for determination of lipase activity. This method is based on the hydrolysis of tributyrin by the enzyme. The butyric acid liberated is determined by titration with NaOH. One NOVO Lipase Unit (LU) is the amount of enzyme which, in a pH-stat and under the standard conditions stated below, liberates titratable butyric acid equivalent to 1 µmol of NaOH per minute.

Standard conditions

| Temperature | 30.0°C |
|---|---|
| pH | 7.0 |
| Reaction time | 20 minutes |
| Substrate | tributyrin |

Further details of this method are described in the leaflet AF 95/4 dated 1982—08—18, which is available on request from NOVO Industri A/S, Bagsvaerd, Denmark.

In a specially preferred embodiment of the enzymatic detergent additive according to the invention, the lipase producing strain of *Fusarium oxysporum* is DSM 2672. The strain *Fusarium sp.* DSM 2672 has been classified as *Fusarium oxysporum* Schlecht. ex Fries, emend. Snyder & Hansen. The morphological properties of the strain DSM 2672 *Fusarium oxysporum* exactly correspond to the description of the species description of *Fusarium oxysporum* in the Genus *Fusarium*, C. Booth, CMI, 1971. The pH activity curve of the lipase has been drawn up, the lipase activity being measured according to AF 95/4-GB, modified by adjustment of the pH value of the substrate to 4, 5, and 6, and 8, 9, and 10 besides the normal pH value of the substrate of 7. Due to the fact that tributyrin is decomposed without lipase at high pH values, the pH curve is corrected for such autodecomposition. Hereby a pH activity optimum of around pH 10 has been found. The stability of the enzyme is excellent over a wide pH interval, inasmuch as more than 80% residual activity after 18 hours can be observed in the pH interval of 4.5—11 at 5°C and in the pH interval of 5—10 at 25°C. Also, the temperature optimum of the lipase activity is around 40°C. The enzyme is stable up to 40°C for 30 minutes, which is very advantageous in relation to the previously mentioned low-temperature washing processes at around 60°C and below.

In a specially preferred embodiment of the enzymatic detergent additive according to the invention, the additive is provided as a non-dusting granulate. These granulates can be produced in several different ways. Reference can be made to GB patent No. 1,362,365 which describes the production of enzyme-containing granulates used as detergent additives by means of an apparatus comprising an extruder and a spheronizer (sold as Marumerizer®), and to US patent No. 4,106,991 which describes the production of enzyme containing granulates used as detergent additives by means of a drum granulator.

In a specially preferred embodiment of the enzymatic detergent additive according to the invention, the additive is provided as a liquid with an enzyme stabilizer. The stabilizer can be propylene glycol or other agents known as stabilizers for enzyme solutions. Liquid detergents exhibit a growing popularity due to the ease of application.

In a specially preferred embodiment of the enzymatic detergent additive according to the invention, the lipase activity is between 20,000 and 100,000 LU/g of additive. In this manner, a convenient lipase activity is generated in the washing solution when the detergent additive is added to the detergent in an amount of 0.2—2 g/100 g of detergent, and when the detergent is added to the washing solution in an amount of 1—5 g of detergent/l of washing solution.

In a specially preferred embodiment of the enzymatic detergent additive according to the invention, the additive contains a proteolytic enzyme besides the lipase. Surprisingly it has been found that the proteolytic detergent additive does not break down the (protein) lipase, either in the additive, in the detergent, or in the washing solution. Thus, the proteolytic and the lipolytic detergent additives are compatible, and it has been found that this detergent additive has a very high cleaning efficiency, resulting in a very high ΔR value. The proteolytic enzyme Alcalase® from NOVO Industri A/S, manufactured microbially by means of *Bacillus licheniformis*, can be used with superior results. The mixed enzymatic additive can be prepared either by mixing a previously prepared granulate of proteinase with a previously prepared granulate of lipase, or by mixing a concentrate of proteinase with a concentrate of lipase and then introducing this mixture into a granulating device, together with the usual granulating aids.

In a specially preferred embodiment of the enzymatic detergent additive according to the invention, the proteolytic activity is between 0.5 and 3.0 Anson Units/g of additive. In this manner, a convenient proteolytic activity is generated in the washing solution when the detergent additive is added to the detergent in an amount of 0.2—2 g/100 g of detergent, and when the detergent is added to the washing solution in an amount of 1—5 g of detergent/l of washing solution.

The second aspect of the invention comprises a detergent with an enzymatic detergent additive, the active component of which is a microbially produced lipase, wherein the enzymatic detergent additive is the enzymatic detergent additive according to the invention.

In a specially preferred embodiment of the enzymatic detergent additive according to the invention, the

**0 130 064**

detergent contains the enzymatic detergent additive according to the invention in an amount of between 0.2 and 2.0% w/w. In this manner, a reasonable balance between enzyme action and the action of the other detergent ingredients is generated.

The third aspect of the invention comprises a washing process in which the detergent used is the detergent according to the invention, and in which the pH is between 7 and 11, and the temperature is below 60°C.

In a specially preferred embodiment of the enzymatic detergent additive according to the invention, the washing solution contains the detergent according to the invention in an amount of between 1 and 5 g/l of washing solution. In this manner, a convenient enzyme activity is generated in the washing solution, i.e. typically between 1,000 and 5,000 LU/l of washing solution. Under these circumstances, very high $\Delta R$ values are obtained for usual washing times, i.e. around 20 minutes.

The invention will be illustrated by the following examples.

Example 1

This example illustrates the production in shake flasks of the active component in the enzymatic detergent additive according to the invention.

The substrate consists of the following ingredients in grams per liter.

| Soy bean meal | 50 |
| Glucose | 50 |
| $KH_2PO_4$ | 2 |
| $Na_2HPO_4$ | 3 |
| Soy oil | 1 |

Sterilization took place at 121°C for 40 minutes. A 500 ml Erlenmeyer flask with 100 ml substrate was inoculated with $10^7$ spores from an agar slant previously inoculated with Fusarium oxysporum DSM 2672. The flasks were shaken at 230 rpm and at 25°—30°C for 2—5 days. The yield was 30—80 LU/ml.

Example 2

This example and examples 3—15 illustrate the production of the active component in the enzymatic detergent additive according to the invention in a 2 litre laboratory fermenter.

A 500 ml Erlenmeyer flask with 260 ml of the following medium was prepared.

| Glucose | 24 g per litre |
| Corn steep liquor | 24 g per litre |
| Soy oil | 3.8 g per litre |
| Calcium carbonate | 3.8 g per litre |
| pH=5.5 before addition of $CaCO_3$ | |

Sterilization took place at 121°C for 40 minutes.

After inoculation with spores from an agar slant in the same manner as indicated in example 1 the flask was agitated at 230 rpm and at 30°C for 2 days. 120 ml of this seed culture were transferred to a two litre fermenter with 1.3 litre medium sterilized for 1 hour at 121°C and of the following composition.

| Glucose | 50 g per litre |
| Soy bean meal | 50 g per litre |
| $KH_2PO_4$ | 2.0 g per litre |
| $Na_2HPO_4 \cdot 2H_2O$ | 3.0 g per litre |
| Soy oil | 10 g per litre |
| Pluronic | 0.3 ml |

Subsequent to the inoculation, aeration (700 ml/min) was initiated. Stirring was also initiated and the velocity thereof was 600 rpm during the first 24 hours, and thereafter it was increased to 800 rpm. The temperature was maintained at 30°C. Fermentation was maintained for 100 hours, during which time the pH value rose from 6.3 to 8.5. The supernatant was analysed, and the lipase activity was found to be 90 LU/ml.

A series of experiments were performed in the same manner as indicated in example 2, except for the temperature, *vide* the following table

| Example No. | 3 | 4 | 5 | 6 | 7 |
|---|---|---|---|---|---|
| Temperature in fermenter, °C | 28 | 30 | 32 | 34 | 36 |
| Lu/ml of supernatant | 60 | 90 | 125 | 140 | 60 |

From the above table it appears that the optimum temperature is around 34°C.

4

In order to investigate the influence of pH two new experiments were performed at 34°C and as indicated in example 6, except for pH: in no one of the two experiments pH was controlled during the first 24 hours of the fermentation in the fermenter; in the first experiment (example 8) the pH was maintained at 6.5 by means of $H_3PO_4$ after the first 24 hours of the fermentation in the fermenter, and in the second experiment (example 9) the pH was not monitored during the entire fermentation time. The results appear from the below indicated table.

| Example No. | | 8 | 9 |
|---|---|---|---|
| Temperature in fermenter, °C | | 34 | 34 |
| pH | after 24 hours maintained at 6.5 | x | |
| | no regulation | | x |
| LU/ml of supernatant | | 180 | 140 |

From the above table it appears that the lipase yield is improved if the pH value is monitored in the manner indicated.

In order to investigate the influence of the degree of agitation three experiments were performed in the same manner as indicated in example 8 except for the degree of agitation: the speed of the agitator was raised from 600 rpm to different levels during the entire fermentation time after the first 24 hours fermentation, *vide* the following table. Also the results appear from the following table.

| Example No | 10 | 11 | 12 |
|---|---|---|---|
| Temperature in fermenter, °C | 34 | 34 | 34 |
| After 24 h pH maintained at 6.5 | x | x | x |
| Speed of stirrer, rpm | 700 | 800 | 900 |
| LU/ml of supernatant | 100 | 180 | 160 |

From the above table it appears that the optimum degree of agitation is around 800 rpm.

In order to investigate the influence of the concentration of soy oil in the fermentation medium three experiments were performed in the same manner as indicated in example 11 (i.e. with optimal values for temperature, pH and degree of agitation), except for the concentration of soy oil, *vide* the following table.

| Example No | 13 | 14 | 15 |
|---|---|---|---|
| Temperature in fermenter, °C | 34 | 34 | 34 |
| After 24 hours pH maintained at 6.5 | x | x | x |
| Speed of stirrer, rpm | 800 | 800 | 800 |
| Soy oil, % | 0 | 1 | 2 |
| LU/ml of supernatant | 50 | 170 | 170 |

Example 16

This example illustrates the production of the active component in the enzymatic detergent additive according to the invention in a pilot plant fermenter.

In an inoculation tank with a volume of 300 l the following medium was prepared.

| | |
|---|---|
| Corn steep liquor | 7.2 kg |
| Soy oil | 1.5 kg |
| Calcium carbonate | 1.5 kg |
| Pluronic | 25 ml |
| Water up to | 290 l |

5

The medium was sterilized at 121°C for 1 hour. A twelve liter solution containing 7.2 kg glucose and 7.2 g citric acid was sterilized at 121°C for 1/2 hour and added to the corn steep liquor mixture. After cooling to 30°C the medium was inoculated with spores of DSM 2672 from a Fernbach flask containing yeast extract, phosphate, magnesium, glucose and agar and which had been incubated at 30°C for 7 days. Aeration (300 l/min) was started immediately, and stirring (250 rpm) was initiated after 20 hours. The temperature was maintained at 30°C and growth was maintained for 29 hours. 30 l of this seed culture were transferred to the main fermenter containing:

| | |
|---|---|
| Soy bean meal | 15 kg |
| KH$_2$PO$_4$ | 0.6 kg |
| Na$_2$HPO$_4$12H$_2$O | 1.2 kg |
| Soy oil | 3 kg |
| Pluronic | 25 ml |
| Water to a volume of 275 liters. | |

The medium was sterilized for 1 hour at 121°C. 15 kg glucose and 15 g citric acid in 25 l water were sterilized separately at 121°C for 30 minutes and added to the soy bean meal mixture.

Following the inoculation, aeration (300 l/min) was started. Stirring was also started, and the velocity thereof was increased to 400 rpm during the first 10 hours of fermentation. The temperature was maintained at 34°C. Fermentation was maintained for 60 hours, during which time the pH value rose from 6.64 to 8, and thereafter the tank was cooled and the mycelium separated by centrifugation. The supernatant liquid was analysed, and the lipase activity was found to be 90 LU/ml.

Example 17

This example illustrates the production of the active component in the enzymatic detergent additive according to the invention in a pilot plant fermenter.

The lipase was prepared by submerged aerobic fermentation of Fusarium oxysporum DSM 2672.

An agar substrate with the following composition was prepared in a Fernbach flask:

| | |
|---|---|
| Yeast extract Difco | 4 g |
| K$_2$HPO$_4$ | 1 g |
| MgSO$_4$ · 7H$_2$O | 0.5 g |
| Glucose | 15 g |
| Distilled water ad. | 1000 ml |
| Agar Merck | 15 g |

The mixture was autoclaved for 40 min. at 120°C (The substrate is named YPG-agar).

The strain DSM 2672 was cultivated at 30°C for one week on an YPG-agar slant. The spores from the slant were suspended in sterilized skim milk, and the suspension was lyophilized in vials. The contents of one lyophilized vial was transferred to the Fernbach flask. The flask was then incubated for one week at 30°C.

A substrate with the following composition was prepared in a 500 liter seed fermenter:

| | |
|---|---|
| CaCO$_3$ | 1.5 kg |
| Glucose | 7.2 kg |
| Corn steep liquor | 7.2 kg |
| Antifoam agent Pluronic® | 30 ml |

Tap water was added to a total volume of around 240 liters. pH was adjusted to around 5.5 before addition of CaCO$_3$. The substrate was steam sterilized in the seed fermenter for 1 hour at 121°C. Final volume before inoculation was around 300 liters.

The Fernbach flask spore suspension was transferred to the seed fermenter. Seed fermentation conditions were:

| | |
|---|---|
| Fermenter type: | Conventional aerated and agitated fermenter with a height/diameter ratio of around 2. |
| Agitation: | 300 rpm (two turbine impellers) |
| Aeration: | 300 normal liter air per minute. |
| Temperature: | 30°C |
| Pressure: | 0.51 bar (0.5 ato). |
| Time: | Around 17 hours. |

In order to prevent excessive foaming reduction in agitation and aeration rate was possible. However, in the seed fermentation described here this possibility was not used. After 1/2—1 day when good growth

6

was obtained, here around 17 hours after inoculation, 25 liters were transferred from the seed fermenter to the main fermenter.

A substrate with the following composition was prepared in a 500 liter main fermenter:

| | |
|---|---|
| Toasted, dehulled soy meal | 39 kg |
| Glucose | 15 kg |
| Soy oil | 3 kg |
| $KH_2PO_4$ | 0.6 kg |
| $Na_2HPO_4 \cdot 12H_2O$ | 1.2 kg |
| Antifoam agent Pluronic® | 30 ml |

Tap water was added to a total volume of around 250 liters. The soy bean meal was suspended in water. pH was adjusted to 8.0 with $Na_2CO_3$, and the temperature was raised to 50°C. Thereafter around 480 Anson Units of Alcalase® L (4 AU/ml) was added to the suspension. The mixture was held for 4 hours at 50°C and pH=8.0 ($Na_2CO_3$ addition) with no aeration, zero bar (ato) and 150—200 rpm agitation. Thereafter the remaining substrate components were added and pH was adjusted to around 6.5 with phosphoric acid. The substrate was steam sterilized in the main fermenter for 1 hour at 121°C. After cooling to 34°C pH was adjusted to around 6.0 with sterilized $Na_2CO_3$ solution (3 kg $Na_2CO_3$ in 20 liters total volume). Final volume before inoculation was around 280 liters. Then 25 liters of seed culture was added.

Fermentation conditions were:

| | |
|---|---|
| Fermenter type: | Conventional aerated and agitated fermenter with a height/diameter ratio of around 2. |
| Agitation: | 150 rpm (0—6 hours) and 400 rpm (6 hours to end) (2 turbine impellers). |
| Aeration: | 200—250 normal liter air per minute (0—22 hours) and 300 normal liter air per minute (22 hours to end). |
| Temperature: | 34°C |
| Pressure: | 1.01 bar (1.0 ato) (0—34 hours) and 0.51 bar (0.5 ato) (34 hours to end) |
| Time: | Around 112 hours. |

In order to prevent excessive foaming it was possible to vary agitation, aeration, and pressure. In the fermentation described here this possibility was used as can be seen from the data given above.

Fermentation pH was kept at around 6.5 with addition of phosphoric acid solution. In the fermentation described here dosage occurred from around 30 fermentation hours to the end. The phosphoric acid solution was prepared in a 500 liter dosage tank as follows: to 20 kg conc. phosphoric acid (80%) tap water was added to a total volume of around 160 liters. The solution was steam-sterilized in the dosing tank for 1 hour at 121°C. Final volume before start of dosage was around 200 liters. Only the amount of phosphoric acid solution required to keep fermentation pH at around 6.5 was added to the main fermenter.

During the fermentation sterilized antifoam agent P2000 (polypropylene glycol) was added to the main fermenter to keep down foam formation. In the fermentation described here 3.5 liters of P2000 was used.

After around 112 fermentation hours the fermentation process was stopped. The main fermenter contained around 315 liters of culture broth and the lipase yield obtained was around 200 LU per gram of broth, measured according to NOVO's LU-assay No 95/5-GB using tributyrin as substrate.

Example 18

The lipase was produced as described in example 17 but the fermentation was carried out in a 2500 liter main fermenter with correspondingly higher substrate amounts and volumes.

Agitation: 250 rpm (two turbine impellers)

To prevent excessive foaming, aeration and pressure were varied as follows:

| | |
|---|---|
| Aeration: | 750 Nl/min air (0—13 hours) |
| | 1200 Nl/min air (13—37 hours) |
| | 1500 Nl/min air (37 hours to end) |
| Pressure: | 0.71 bar (0.7 ato) (0—25 hours) |
| | 0.51 bar (0.5 ato) (25 hours to end) |

No antifoam agent P2000 was added in this fermentation. At the end of fermentation the fermenter contained around 1800 liters of culture broth and the lipase yield obtained was around 125 LU per gram of broth.

7

Example 19

The lipase was produced as described in example 17 but the fermentation was carried out in a 2500 liter main fermenter with correspondingly higher amounts and volumes of substrate and the Alcalase treatment of the soy meal was omitted. 200 kg soy meal was used and the volume before inoculation was 1400 liters. The substrate was steam sterilized in the main fermenter for 1.5 hours at 123°C.

agitation: 250 rpm (two turbine impellers) to prevent excessive foaming, aeration and pressure were varied as follows:

aeration:
750 Nl/min of air (0—3 hours)
900 Nl/min of air (3—11 hours)
1100 Nl/min of air (11—24 hours)
1300 Nl/min of air (24—27 hours)
1500 Nl/min of air (27 hours to end)

pressure:
1.01 bar (1.0 ato) (0—27 hours)
0.76 bar (0.75 ato) (27—40 hours)
0.51 bar (0.5 ato) (40 hours to end)

No antifoam agent P2000 was added in this fermentation. At the end of the fermentation the fermenter contained around 1400 liters of culture broth and the lipase yield obtained was around 198 LU per gram broth. Fermentation time was around 140 hours.

Example 20

The lipase was produced as described in example 17 but the fermentation was carried out in a 2500 liter main fermenter with correspondingly higher substrate amounts and volumes, and the Alcalase treatment of the soy meal was omitted. 170 kg soy meal was used and the volume before inoculation was 1400 liters. The substrate was steam sterilized in the main fermenter for 1.5 hours at 123°C.

agitation: 250 rpm (two turbine impellers) to prevent excessive foaming, aeration and pressure were varied as follows:

aeration:
750 Nl/min of air (0—11 hours)
1150 Nl/min of air (11—19 hours)
1300 Nl/min of air (19—27 hours)
1500 Nl/min of air (27 hours to end)

pressure:
1.01 bar (1.0 ato) (0—27 hours)
0.51 bar (0.5 ato) (27 hours to end)

This fermentation used 0.5 liter of antifoam agent P2000. At the end of the fermentation the fermenter contained around 1400 liters of culture broth and the lipase yield obtained was around 221 LU per gram broth. Fermentation time was around 144 hours.

Example 21

The lipase was produced as described in example 17 but the soy meal concentration in the main fermenter substrate was reduced to 5% and the Alcalase treatment of the soy meal was omitted.

To prevent excessive foaming, agitation was varied as follows:

Agitation:
0 rpm (0—22 hours)
100—150 rpm (22—33 hours)
300 rpm (33 hours to end)

No antifoam agent P2000 was added in this fermentation. At the end of fermentation the fermenter contained around 310 liters of culture broth and the lipase yield obtained was around 146 LU per gram of broth.

Example 22

The lipase was produced as described in example 17 but the soy meal concentration in the main fermenter substrate was reduced to 5%, Alcalase treatment was omitted, soy oil was removed from the initial fermentation medium and instead sterilized soy oil was added to the main fermenter 1 liter at a time at 48, 60, 72, and 84 fermentation hours, totalling 4 liters of soy oil.

To prevent excessive foaming, agitation, aeration and pressure was varied as follows:

| Agitation: | 200—300 rpm (0—24 hours) |
| | 400 rpm (24 hours to end) |
| Aeration: | 150 Nl/min air (0—17 hours) |
| | 275 Nl/min air (17—25 hours) |
| | 300 Nl/min air (25 hours to end) |
| Pressure: | 0.91—1.01 bar (0.9—1.0 ato) (0—33 hours) |
| | 0.51 bar (0.5 ato) (33 hours to end) |

This fermentation used 2 liters of antifoam agent P2000.

At the end of fermentation the fermenter contained around 285 liters of culture broth and the lipase yield obtained was around 113 LU per gram of broth.

Example 23

The culture broth from example 19 was adjusted to pH 8.0 with sodium hydroxide and flocculated with 1% calcium chloride, 1% Servamine KZA 346 (Servo), 0.03% Superfloc A-130 (American Cyanamid) and 0.5% Triton X-100 (Rohm and Haas).

The flocculated culture broth was centrifuged on a disc centrifuge (Westfalia SAMS).

The centrifugate was adjusted to pH 4.5 with acetic acid. At this pH a precipitate was formed. The precipitate was separated from the supernantant by centrifugation (Westfalia SAMS) and then treated with Triton X-100. The treated precipitate was then diluted with water and centrifuged again.

The centrifugate from the two centrifugations were mixed and the sludge from the second centrifugation step was discarded.

The centrifugate was diafiltered (DDS-module GR6oP membranes), and afterwards ultrafiltered and concentrated.

The UF-concentrate was filtered on a polypropylene cloth with Hy-flo-supercell (Johns Manville) as filter aid. The filtrate was then adjusted to pH 8.0 with sodium hydroxide and filtered on Supra 100 filter sheets (Seitz) and at last germ filtered on·Supra EKS (Seitz).

The germ filtrate was precipitated with an equal quantity of acetone and washed with pure acetone.

The precipitate was dried in a vacuum chamber. The dried concentrate obtained had an activity of about 90,000 LU/g.

Example 24

Washing efficiency of a Fusarium oxysporum lipase of the invention compared to other lipases.

| Test material: | EMPA 101 (olive oil/cotton) |
| Detergent solution: | Detergent A*), 5 g/l 10° dH water |
| Washing machine: | TERG-O-TOMETER |
| Washing programme: | 40°C for 20 minutes |
| No of Swatches: | 9/900 ml washing solution |
| Lipase: | Fusarium oxysporum lipase |
| | Asp. niger, AMANO AP6 |
| | Candida cylindracea, Sigma |
| | Mucor miehei, SP 225 |
| Lipase conc.: | 0, 750, 1500, 2250, 3000 LU/l |

The Fusarium oxysporum lipase activity units were introduced as suitable volumes of the supernatant from the centrifugation of the culture broth, the production of which is described in example 1.

The washing efficiency is expressed as R=Remission value, measured by the Elrepho photometer, filter R 46, average of 2 readings on 9 swatches.

*) Detergent A has the following composition:

| Linear alkyl benzene sulphonate (LAS, anionic surfactant) | 17% |
| Nonyl phenol ethoxylate, EO=12 (non ionic surfactant) | 3% |
| Sodium tripolyphosphate (STPP) | 30% |
| Sodium silicate | 4% |
| Sodium carbonate | 3% |
| Sodium sulphate | 37% |
| Water | 6% |

In the table the results appear as R and $\Delta R = R - R_0$ in which $R_0$ is the remission value of swatches washed without lipase, and R is the remission value of swatches washed with lipase.

9

| Lipase | Lipase concentration, LU/l | | | | | | | |
| | 750 | | 1500 | | 2250 | | 3000 | |
| | R | ΔR | R | ΔR | R | ΔR | R | ΔR |
| --- | --- | --- | --- | --- | --- | --- | --- | --- |
| None, $R_0$ | 46.1 | 0.0 | 46.1 | 0.0 | 45.1 | 0.0 | 45.2 | 0.0 |
| Fusarium oxysporum lipase | — | — | 53.2 | 7.1 | 53.4 | 8.3 | 54.0 | 8.8 |
| A. niger, AMANO AP6 | 47.0 | 0.9 | 48.2 | 2.1 | 48.6 | 3.5 | 49.8 | 4.6 |
| C. cylindracea, sigma | 46.2 | 0.1 | 46.4 | 0.3 | 46.4 | 1.3 | 45.6 | 0.4 |
| M. miehei, SP 225 | 49.6 | 3.5 | 48.1 | 2.0 | 49.9 | - 4.8 | 49.6 | 4.4 |

Example 25

In order to demonstrate the lipolytic effect on other textiles than cotton, test material was prepared on polyester/cotton and acrylic as well.

Test soiling:    10% olive oil
1.5% emulsifier
0.4% earthen colour
0.3% indian ink
87.8% water

The solution was emulsified in a Rannie homogenizer. Pieces of cotton, polyester/cotton and acrylic were immersed in the homogenized solution. The excess soiling was squeezed off and the pieces of cloth were dried in a spindrier for 30 minutes.

Washing conditions

Test material:    Olive oil on:    I cotton
II polyester/cotton
III acrylic

Detergent solution:    detergent B**), 3.25 g/l, 10°C dH water
detergent C***), 1 g/l, 10° dH water
Washing machine:    Launder-O-Meter
Washing programme:    heating from 15°C to 40°C, heating time
37 minutes, washing at 40°C for 12 minutes
No of swatches:    3/300 ml washing solution
Lipase:    Fusarium oxysporum lipase
Mucor miehei, SP 225
C. cylindracea, sigma
Pancreatic lipase, sigma
Lipase concentration:    varied

The Fusarium oxysporum lipase units were introduced as suitable quantities of a freeze dried powder produced in the following manner. A fermentation in a 1 liter laboratory flask was carried out in much the same manner as indicated in example 1. The culture broth was centrifuged, concentrated by ultrafiltration and freeze dried.

**) Detergent B has the following composition:

Linear alkyl benzene sulphonate (LAS,
    anionic surfactant)    34%
Nonyl phenol ethoxylate, EO=12 (non
    ionic surfactant)    3%
Sodium tripolyphosphate (STPP)    62%
Carboxymethylcellulose (CMC)    1%

***) Detergent C is 100% nonyl phenol ethoxylate, EO=12 (non ionic surfactant).

The washing efficiency is expressed as R=Remission value, measured by the Elrepho photometer, filter R 46, average of 2 readings on 3 swatches.

In the table, $R_0$ and ΔR have the same meaning as indicated in Example 24.

Active component in enzymatic detergent additive according to the invention.

| Test material | Detergent B Lipase concentration, LU/l | | | | | Detergent C Lipase concentration, LU/l | | | | |
|---|---|---|---|---|---|---|---|---|---|---|
| | 0 $R_0$ | 84 $\Delta R$ | 167 $\Delta R$ | 333 $\Delta R$ | 1000 $\Delta R$ | 0 $R_0$ | 84 $\Delta R$ | 167 $\Delta R$ | 333 $\Delta R$ | 1000 $\Delta R$ |
| cotton | 32.2 | 2.8 | 6.7 | 7.9 | 10.7 | 27.9 | 4.9 | 4.7 | 7.9 | 11.4 |
| polyester/cotton | 44.8 | 2.9 | 3.5 | 3.9 | 3.6 | 31.6 | 4.4 | 5.0 | 4.6 | 5.2 |
| acrylic | 56.7 | 3.9 | 5.6 | 7.0 | 6.7 | 33.7 | 5.8 | 10.5 | 11.0 | 10.2 |

Mucor miehei lipase

| Test material | Detergent B Lipase concentration, LU/l | | | | | Detergent C Lipase concentration, LU/l | | | | |
|---|---|---|---|---|---|---|---|---|---|---|
| | 0 $R_0$ | 750 $\Delta R$ | 3000 $\Delta R$ | 6000 $\Delta R$ | 10000 $\Delta R$ | 0 $R_0$ | 750 $\Delta R$ | 3000 $\Delta R$ | 6000 $\Delta R$ | 10000 $\Delta R$ |
| cotton | 35.3 | 4.1 | 4.8 | 7.5 | 8.8 | 25.4 | 1.9 | 2.0 | 4.3 | 4.9 |
| polyester/cotton | 44.3 | 2.3 | 3.1 | 3.7 | 1.6 | 31.4 | 1.8 | 2.0 | 1.8 | 1.0 |
| acrylic | 54.4 | 3.6 | 4.4 | 5.7 | 5.7 | 33.8 | 4.4 | 5.8 | 6.5 | 6.3 |

C. cylindracea, pancreatic lipase                               Test material: cotton

| Detergent | C. cylindracea lipase Lipase concentration, LU/l | | | | | Pancreatic lipase Lipase concentration, LU/l | | | | |
|---|---|---|---|---|---|---|---|---|---|---|
| | 0 $R_0$ | 250 $\Delta R$ | 500 $\Delta R$ | 750 $\Delta R$ | 3000 $\Delta R$ | 0 $R_0$ | 750 $\Delta R$ | 3000 $\Delta R$ | 6000 $\Delta R$ | 10000 $\Delta R$ |
| B | 36.4 | 0.0 | 0.0 | 1.1 | 0.0 | 34.5 | 2.7 | 2.2 | 3.0 | 3.8 |
| C | 26.0 | 0.0 | 0.0 | 0.4 | 0.5 | 24.4 | 3.4 | 3.8 | 3.5 | 3.6 |

Example 26

This example demonstrates the compatibility of a proteolytic enzyme and the lipase in the enzymatic detergent additive according to the invention. The test material was cotton soiled as indicated in Example 25.

Washing conditions

| | |
|---|---|
| Test material: | Cotton soiled as indicated above. |
| Detergent solution: | Detergent B 3.25 g/l, 10° dH water |
| | Detergent C 1.0 g/l, 10° dH water |
| Washing machine: | Launder-O-meter |
| Washing programme: | Heating from 15°C to 40°C, heating time |
| | 37 minutes, washing at 40°C for 12 minutes. |
| No of swatches: | 3/300 ml washing solution |
| Enzymes: | Fusarium oxysporum lipase |
| | Alcalase 2.0 T |
| Lipase concentration: | 0; 250; 500; 750; 3000 LU/l |
| Proteolytic concn.: | 0; 0.06 or 0.01; 0.02; 0.04; 0.08 AU/l |

The Fusarium oxysporum lipase units were introduced as suitable volumes of the supernatant from the centrifugation of the culture broth, the production of which is described in example 1.

The proteolytic activity is measured in Anson Units/l (AU/l) and is determined according to the modified Anson method described in NOVO ENZYME INFORMATION 1B No. 058 e-GB (the original Anson method is described in J. Gen. Physiol., 22, 79—89 (1939)).

The washing efficiency is expressed as indicated in Example 25.

The meaning of the symbols in the table is the same as indicated in Example 24.

The results appear from the following table.

| Lipase concentration LU/l | Proteinase concentration AU/l | Remission value | Detergent B | Detergent C |
|---|---|---|---|---|
| 0 | 0 | $R_0$ | 35.1 | 27.0 |
| 250 | 0 | $\Delta R$ | 3.5 | 2.7 |
| 500 | 0 | $\Delta R$ | 3.8 | 3.2 |
| 750 | 0 | $\Delta R$ | 5.0 | 3.5 |
| 3000 | 0 | $\Delta R$ | 7.5 | 6.5 |
| 250 | 0.06 | $\Delta R$ | 2.8 | 3.5 |
| 500 | 0.06 | $\Delta R$ | 3.4 | 5.1 |
| 750 | 0.06 | $\Delta R$ | 3.8 | 5.6 |
| 3000 | 0.06 | $\Delta R$ | 5.4 | 6.4 |
| 0 | 0.01 | $\Delta R$ | 1.5 | 1.9 |
| 0 | 0.02 | $\Delta R$ | 1.2 | 2.4 |
| 0 | 0.04 | $\Delta R$ | 1.1 | 1.4 |
| 0 | 0.08 | $\Delta R$ | 1.8 | 1.5 |

Example 27

This example illustrates the superior washing properties of the lipolytic detergent additive according to the invention in comparison to known lipolytic detergent additives.

Two sets of washing conditions were used:

1: 0—3000 LU/l, EMPA 101 Terg-O-tometer: 40°C for 20 minutes Detergent ALL+$Na_2SO_4$: 3.75+1.25 g/l, 10° dH, pH=9.5

2: 0—3000 LU/l, 10% olive oil/cotton Launder-O-meter: European wasc 40°C Berol wash (a nonionic tenside) 1.0 g/l; NOVO detergent 3.25 g/l, 10° dH, pH 8.5/9.5

. The 3.25 g NOVO detergent consists of 1 g LAS 2 g STPP, 0.05 g CMC, 0.1 g Berol wasc.

The following table shows the washing effect as $\Delta R$ values for different prior art lipolytic detergent additives in comparison.to the lipolytic detergent additive according to the invention. More than one value with a slash in between indicates that two measurements were made.

| Microorganism or origin | Company or commercial name | Washing conditions 1 | Washing conditions 2 |
|---|---|---|---|
| A. niger | NOVO | 3 | |
| | Lipase A | 0 | |
| | AMANO AP G | 5 | |
| Rhizopus rhizopodiformis | NOVO | 0 | |
| C. cylindracea | SIGMA | 0 | 0/0 |
| | Lipase MY | 0 | |
| | Lipase OF | | 0/0 |
| M. miehei | NOVO | 8 | 5/8 |
| M. javanicus | AMANO MAP-10 | 0 | 2/3 |
| F. oxysporum | NOVO | 10 | 10/8 |
| Bacillus circulans | NOVO | | 6/5 (100 LU/l) |
| Geotrichum sp. | NOVO | 0 | |
| Pancreatin | SIGMA | | 4/4 |

The superiority of lipolytic detergent additive based on Fusarium oxysporum in comparison to the prior art lipolytic detergent additives clearly appears from the table.

# 0 130 064

Example 28

This example illustrates the washing characteristics of lipases from different lipase producing Fusarium oxysporum strains.

The test material was acrylic fabric soiled in the following manner

Test soiling:
3% olive oil
1.5% emulsifier
0.4% earthen colour
0.3% indian ink
87.8% water

The solution was emulsified in a Rannie homogenizer. Pieces of acrylic were immersed in the homogenized solution. The excess soiling was squeezed off and the pieces of cloth were dried in a spindrier for 30 minutes.

Experiment I

Presoaking: 5 swatches ($5 \times 10$ cm) are soaked for 2 hours at room temperature in a solution containing 50 ml diluted fermentation broth and 5 ml of solution A. Solution A: Berol wasc (nonyl phenol ethoxylate) in 10° dH water (0.00603% $MgCl_2 \cdot 6H_2O$, 0.0165% $CaCl_2$ and 0.035% $NaHCO_3$ in deionized water).

Wash: The swatches are rinsed for 10 minutes after soaking and washed in a Terg-O-tometer at 30°C for 10 minutes in a detergent solution containing 1.33 g TOP/litre of water of 10° dH.

The swatches are rinsed in tap water for 10 minutes after the washing has been finished and dried.

The detergency is expressed as $\Delta R$.

Results

| | $R_0$ | $\Delta R$ | | | | |
|---|---|---|---|---|---|---|
| Enzyme dosage LU/l of presoak solution 0 | | 750 | 1500 | 3000 | 4500 | 9000 |
| C 597 | | 5.7 | 6.2 | 6.7 | 8.4 | 8.4 |
| | 30.9 | | | | | |
| A 1714 | | 3.9 | 5.1 | 6.0 | 6.7 | — |

Experiment II

A procedure similar to the one described above (Experiment I) was used to test culture broth from some more lipase producing Fuasrium oxysporum strains.

Results

| | $R_0$ | $\Delta R$ | | | | |
|---|---|---|---|---|---|---|
| Enzyme dosage LU/l of presoak solution 0 | | 750 | 1500 | 3000 | 4500 | 9000 |
| C 597 | | 0.9 | 3.2 | 3.7 | 4.9 | |
| A 1714 | | 2.1 | 3.6 | 3.3 | 5.1 | |
| A 1755 | 36.8 | 3.5 | 2.8 | 3.1 | 5.4 | |
| A 1756 | | 3.0 | 4.7 | 4.7 | 6.1 | |
| A 1760 | | 2.0 | 3.7 | 5.3 | 5.9 | |

Example 29

This example illustrates the fact that some Fusarium oxysporum strains are lipase producers according to the definition put forward in this specification, whereas other Fusarium oxysporum strains are not lipase producers according to the definition put forward in this specification.

The substrate for lipase production consists of the following ingredients in grams/l:

| | |
|---|---|
| Soy bean meal | 45 |
| Glucose | 70 |
| $KH_2PO_4$ | 2 |
| $Na_2HPO_4$ | 3 |
| Soy oil | 5 |

13

Sterilization took place at 121°C for 40 minutes. Eleven 500 ml Erlenmeyer flasks, each with 100 ml of substrate, were inoculated with $10^7$ spores from agar slants previously inoculated with eleven Fusarium species. The flasks were shaken at 230 rpm and at 30°C for 5 days. The yields are shown below:

| Scientific identifying designation of strain | Internal identification designation | Official identification designation | Yield, LU/ml |
|---|---|---|---|
| Fusarium oxysporum Schlecht. | C597 | DSM 2672 | 122.5 |
| f. sp. vasinfectum | A1714 | ATCC 7808 | 18.5 |
| f. sp. chrysanthemi | | CBS 127.81 | 1.8 |
| f. sp. cyclaminis | | CBS 159.57 | 5.1 |
| f. sp. gladioli | A1755 | CBS 620.72 | 15.2 |
| f. sp. lycopersici | A1756 | CBS 645.78 | 14.6 |
| f. sp. narcissi | | CBS 196.65 | 7.6 |
| f. sp. opunciarum | | CBS 743.79 | 3.3 |
| f. sp. passiflora | | CBS 744.79 | 7.9 |
| f. sp. perniciosum | A1760 | CBS 794.70 | 10.9 |
| f. sp. lini | | ATCC 10960 | 1.3 |

All strains indicated in this specification are available to the public.

The features disclosed in the foregoing description, and/or in the following claims may, both separately and in any combination thereof, be material for realising the invention in diverse forms thereof.

The words NOVO, MARUMERIZER, ALCALASE, PLURONIC, DIFCO, MERCK, SERVAMINE, SUPERFLOC, TRITON, HY-FLO-SUPERCELL, SUPRA, TERG-O-TOMETER, LAUNDER-O-METER, SIGMA, AMANO, ALL and BEROL WASC are Trade Marks.

**Claims for the Contracting States: BE, CH, DE, FR, GB, IT, LI, NL**

1. Detergent comprising an enzymic additive, the active component of which is a microbially produced lipase, characterised in that the lipase is a lipase produced by means of a lipase producing strain of *Fusarium oxysporum*, which produces more than 10 LU/ml under the standard fermentation conditions.

2. Detergent according to Claim 1, wherein the lipase producing strain of *Fusarium oxysporum* is DSM 2672.

3. Detergent according to Claim 1 or 2, wherein the additive is in the form of a non-dusting granulate.

4. Detergent according to Claim 1 or 2, wherein the additive is in the form of a liquid with an enzyme stabilizer.

5. Detergent according to Claim 3 or 4, wherein the lipase activity is in the range of from 20,000 to 100,000 LU/g of additive.

6. Detergent according to any one of the preceding claims, wherein the additive contains, besides the lipase, a proteolytic enzyme.

7. Detergent according to Claim 6, wherein the proteolytic activity is in the range of from 0.5 to 3.0 Anson Units/g of additive.

8. Detergent according to any one of the preceding claims, wherein the detergent contains the enzymatic detergent additive in an amount of from 0.2 to 2.0% w/w.

9. An enzymic detergent additive suitable for use in preparing a detergent in accordance with Claim 3, in which additive the active component is a microbially produced lipase, characterised in that the lipase is a lipase produced by means of a lipase producing strain of *Fusarium oxysporum*, which produces more than 10 LU/ml under the standard fermentation conditions, in the form of a non-dusting granulate.

10. An enzymic detergent additive suitable for use in preparing a detergent in accordance with Claim 4, in which additive the active component is a microbially produced lipase, characterised in that the lipase is a lipase produced by means of a lipase producing strain of *Fusarium oxysporum*, which produces more than 10 LU/ml under the standard fermentation conditions, in the form of a liquid with an enzyme stabiliser.

11. The use of a lipase producible by means of a lipase producing strain of *Fusarium oxysporum*, which

14

produces more than 10 LU/ml under the standard fermentation conditions, as an active component of an enzymic detergent additive.

12. The use according to Claim 11, wherein the lipase producing strain of *Fusarium oxysporum* is DSM 2672.

13. Washing process, characterised in that the detergent is a detergent according to any one of Claims 1 to 8, the pH is in the range of from 7 to 11, and the temperature is below 60°C.

14. Washing process according to Claim 13, wherein the washing solution contains the detergent in an amount in the range of from 1 to 5 g per litre of washing solution.

15. The use of a detergent according to any one of Claims 1 to 8 for washing.

**Claims for the Contracting State: AT**

1. In a method of preparing a detergent comprising an enzymic additive, the active component of which is a microbially produced lipase, the use of a lipase produced by means of a lipase producing strain of *Fusarium oxysporum*, which produces more than 10 LU/ml under the standard fermentation conditions.

2. A method according to Claim 1, wherein the lipase producing strain of *Fusarium oxysporum* is DSM 2672.

3. A method according to Claim 1 or 2, wherein the additive is in the form of a non-dusting granulate.

4. A method according to Claim 1 or 2, wherein the additive is in the form of a liquid with an enzyme stabilizer.

5. A method according to Claim 3 or 4, wherein the lipase activity is in the range of from 20,000 to 100,000 LU/g of additive.

6. A method according to any one of the preceding claims, wherein the additive contains, besides the lipase, a proteolytic enzyme.

7. A method according to Claim 6, wherein the proteolytic activity is in the range of from 0.5 to 3.0 Anson Units/g of additive.

8. A method according to any one of the preceding claims, wherein the detergent contains the enzymatic detergent additive in an amount of from 0.2 to 2.0% w/w.

9. The use of an enzymic detergent additive in preparing a detergent in accordance with the method of Claim 3, in which additive the active component is a microbially produced lipase, characterised in that the lipase is a lipase produced by means of a lipase producing strain of *Fusarium oxysporum*, which produces more than 10 LU/ml under the standard fermentation conditions, in the form of a non-dusting granulate.

10. The use of an enzymic detergent additive suitable for use in preparing a detergent in accordance with Claim 4, in which additive the active component is a microbially produced lipase, characterised in that the lipase is a lipase produced by means of a lipase producing strain of *Fusarium oxysporum*, which produces more than 10 LU/ml under the standard fermentation conditions, in the form of a liquid with an enzyme stabiliser.

11. The use of a lipase producible by means of a lipase producing strain of *Fusarium oxysporum*, which produces more than 10 LU/ml under the standard fermentation conditions, as an active component of an enzymic detergent additive.

12. The use according to Claim 11, wherein the lipase producing strain of *Fusarium oxysporum* is DSM 2672.

13. Washing process, characterised in that the detergent is a detergent prepared according to any one of Claims 1 to 8, the pH is in the range of from 7 to 11, and the temperature is below 60°C.

14. Washing process according to Claim 13, wherein the washing solution contains the detergent in an amount in the range of from 1 to 5 g per litre of washing solution.

15. The use of a detergent prepared according to any one of Claims 1 to 8 for washing.

**Patentansprüche für die Vertragsstaaten: BE, CH, DE, FR, GB, IT, LI, NL**

1. Waschmittel mit einem enzymatischen Zusatz, deren aktive Komponente eine mikrobiell produzierte Lipase ist, dadurch gekennzeichnet, daß die Lipase eine mittels eines Lipase erzeugenden Stammes von *Fusarium oxysporum*, die unter üblichen Fermentationsbedingungen mehr als 10 LU/ml erzeugt, ist.

2. Waschmittel nach Anspruch 1, wobei der Lipase erzeugende Stamm von *Fusarium oxysporum* DSM 2672 ist.

3. Waschmittel nach Anspruch 1 oder Anspruch 2, wobei das Waschmittel in der Form eines nicht-staubenden Granulats ist.

4. Waschmittel nach Anspruch 1 oder 2, wobei das Additiv in der Form einer Flüssigkeit mit einem Enzymstabilisierer ist.

5. Waschmittel nach Anspruch 3 oder Anspruch 4, wobei die Lipaseaktivität in dem Bereich von 20.000 bis 100.000 LU/g des Additivs liegt.

6. Waschmittel nach einem der vorangehenden Ansprüche, wobei das Waschmittel neben der Lipase ein proteolytisches Enzym beinhaltet.

7. Waschmittel nach Anspruch 6, wobei die proteolytische Aktivität im Bereich von 0,5 bis 3,0 Anson-Einheiten/g des Additivs liegt.

8. Waschmittel nach einem der vorangehenden Ansprüche, wobei das Waschmittel das enzymatische Waschmitteladditiv in einer Menge von 0,2 bis 2,0% Gewichts-% enthält.

9. Enzymatisches Waschmitteladditiv, geeignet zur Verwendung bei der Herstellung eines Waschmittels nach Anspruch 3, wobei die aktive Komponente in dem Additiv eine mikrobiell erzeugte Lipase ist, dadurch gekennzeichnet, daß die Lipase eine mittels eines Lipase erzeugenden Stammes von *Fusarium oxysporum*, der unter üblichen Fermentationsbedingungen mehr als 10 LU/ml erzeugt, erzeugte Lipase ist, in der Form eines nicht-staubenden Granulats.

10. Enzymatisches Waschmitteladditiv, geeignet zur Verwendung bei der Herstellung eines Waschmittels nach Anspruch 4, wobei die aktive Komponente in dem Additiv eine mikrobiell erzeugte Lipase ist, dadurch gekennzeichnet, daß die Lipase eine mittels eines Lipase erzeugenden Stammes von *Fusarium oxysporum*, der unter üblichen Fermentationsbedingungen mehr als 10 LU/ml erzeugt, erzeugte Lipase ist, in der Form eines licht-staubenden Granulats.

11. Verwendung einer mittels eines Lipase erzeugenden Stammes von *Fusarium oxysporum*, der unter üblichen Fermentationsbedingungen mehr als 10 LU/ml erzeugt, erzeugten Lipase als aktive Komponente eines enzymatischen Waschmitteladditivs.

12. Verwendung nach Anspruch 11, wobei der Lipase erzeugende Stamm von *Fusarium oxysporum* DSM 2672 ist.

13. Waschverfahren, dadurch gekennzeichnet, daß das Waschmittel ein Waschmittel nach einem der Ansprüche 1 bis 8 ist, wobei der pH-Wert in dem Bereich von 7 bis 11 liegt und die Temperatur geringer als 60°C ist.

14. Waschverfahren nach Anspruch 13, wobei die Waschlösung das Waschmittel in einem Bereich von 1 bis 5 g pro Liter der Waschlösung aufweist.

15. Verwendung eines Waschmittels nach einem der Ansprüche 1 bis 7 zum Waschen.

**Patentansprüche für den Vertragsstaat: AT**

1. Verfahren zur Herstellung eines Waschmittels mit einem enzymatischen Zusatz, deren aktive Komponente eine mikrobiell produzierte Lipase ist, dadurch gekennzeichnet, daß die Lipase eine mittels eines Lipase erzeugenden Stammes von *Fusarium oxysporum*, die unter üblichen Fermentationsbedingungen mehr als 10 LU/ml erzeugt, ist.

2. Verfahren zur Herstellung eines Waschmittels nach Anspruch 1, wobei der Lipase erzeugende Stamm von *Fusarium oxysporum* DSM 2672 ist.

3. Verfahren zur Herstellung eines Waschmittels nach Anspruch 1 oder Anspruch 2, wobei das Waschmittel in der Form eines nicht-staubenden Granulats ist.

4. Verfahren zur Herstellung eines Waschmittels nach Anspruch 1 oder 2, wobei das Additiv in der Form einer Flüssigkeit mit einem Enzymstabilisierer ist.

5. Verfahren zur Herstellung eines Waschmittels nach Anspruch 3 oder Anspruch 4, wobei die Lipaseaktivität in dem Bereich von 20.000 bis 100.000 LU/g des Additivs liegt.

6. Verfahren zur Herstellung eines Waschmittels nach einem der vorangehenden Ansprüche, wobei das Waschmittel neben der Lipase ein proteolytisches Enzym beinhaltet.

7. Verfahren zur Herstellung eines Waschmittels nach Anspruch 6, wobei die proteolytische Aktivität im Bereich von 0,5 bis 3,0 Anson-Einheiten/g des Additivs liegt.

8. Verfahren zur Herstellung eines Waschmittels nach einem der vorangehenden Ansprüche, wobei das Waschmittel das enzymatische Waschmitteladditiv in einer Menge von 0,2 bis 2,0% Gewichts-% enthält.

9. Verwendung eines enzymatisches Waschmitteladditivs, zur Verwendung bei der Herstellung eines Waschmittels nach dem Verfahren von Anspruch 3, wobei die aktive Komponente in dem Additiv eine mikrobiell erzeugte Lipase ist, dadurch gekennzeichnet, daß die Lipase eine mittels eines Lipase erzeugenden Stammes von *Fusarium oxysporum*, der unter üblichen Fermentationsbedingungen mehr als 10 LU/ml erzeugt, erzeugte Lipase ist, in der Form eines nicht-staubenden Granulats.

10. Verwendung eines enzymatischen Waschmitteladditivs, geeignet zur Verwendung bei der Herstellung eines Waschmittels nach Anspruch 4, wobei die aktive Komponente in dem Additiv eine mikrobiell erzeugte Lipase ist, dadurch gekennzeichnet, daß die Lipase eine mittels eines Lipase erzeugenden Stammes von *Fusarium oxysporum*, der unter üblichen Fermentationsbedingungen mehr als 10 LU/ml erzeugt, erzeugte Lipase ist, in der Form eines nichtstaubenden Granulats.

11. Verwendung einer mittels eines Lipase erzeugenden Stammes von *Fusarium oxysporum*, der unter üblichen Fermentationsbedingungen mehr als 10 LU/ml erzeugt, erzeugten Lipase als aktive Komponente eines enzymatischen Waschmitteladditivs.

12. Verwendung nach Anspruch 11, wobei der Lipase erzeugende Stamm von *Fusarium oxysporum* DSM 2672 ist.

13. Waschverfahren, dadurch gekennzeichnet, daß das Waschmittel ein Waschmittel nach einem der Ansprüche 1 bis 8 hergestellt ist, wobei der pH-Wert in dem Bereich von 7 bis 11 liegt und die Temperatur geringer als 60°C ist.

14. Waschverfahren nach Anspruch 13, wobei die Waschlösung das Waschmittel in einem Bereich von 1 bis 5 g pro Liter der Waschlösung aufweist.

15. Verwendung eines nach einem der Ansprüche 1 bis 8 hergestellten Waschmittels zum Waschen.

## 0 130 064

**Revendications pour les Etats Contractants: BE, CH, DE, FR, GB, IT, LI, NL**

1. Détergent comprenant un additif enzymatique, dont le composant actif est une lipase produite par voie microbiologique, caractérisé en ce que la lipase est une lipase produite à l'aide d'une souche productrice de lipase de *Fusarium oxysporum*, qui produit plus de 10 UL/ml dans les conditions standard de fermentation.

2. Détergent selon la revendication 1, dans lequel la souche productrice de lipase de *Fusarium oxysporum* est DSM 2672.

3. Détergent selon la revendication 1 ou 2, dans lequel l'additif est sous forme de granulés ne formant pas de poussière.

4. Détergent selon la revendication 1 ou 2, dans lequel l'additif est sous forme d'un liquide avec un stabilisant enzymatique.

5. Détergent selon la revendication 3 ou 4, dans lequel l'activité lipasique est dans la gamme de 20 000 à 100 000 UL/g d'additif.

6. Détergent selon l'une quelconque des revendications précédentes, dans lequel l'additif contient en plus de la lipase une enzyme protéolytique.

7. Détergent selon la revendication 6, dans lequel l'activité protéolytique est dans la gamme de 0,5 à 3,0 unités Anson/g d'additif.

8. Détergent selon l'une quelconque des revendications précédentes, lequel détergent contient l'additif enzymatique pour détergent en une quantité de 0,2 à 2,0% p/p.

9. Additif enzymatique pour détergent approprié à l'utilisation dans la préparation d'un détergent selon la revendication 3, dans lequel additif le composant actif est une lipase produite par voie microbiologique, caractérisé en ce que la lipase est une lipase, produite à l'aide d'une souche productrice de lipase de *Fusarium oxysporum* qui produit plus de 10 UL/ml dans les conditions standard de fermentation, sous forme de granulès ne formant pas de poussière.

10. Additif enzymatique pour détergent approprié à l'utilisation dans la préparation d'un détergent selon la revendication 4, dans lequel additif le composant actif est une lipase produite par voie microbiologique, caractérisé en ce que la lipase est une lipase, produite à l'aide d'une souche productrice de lipase de *Fusarium oxysporum* qui produit plus de 10 UL/ml dans les conditions standard de fermentation, sous forme d'un liquide avec un stabilisant enzymatique.

11. Utilisation d'une lipase, pouvant être produite à l'aide d'une souche productrice de lipase de *Fusarium oxysporum* qui produit plus de 10 UL/ml dans les conditions standard de fermentation, comme composant actif d'un additif enzymatique pour détergent.

12. Utilisation selon la revendication 11, dans laquelle la souche productrice de lipase de *Fusarium oxysporum* est DSM 2672.

13. Procédé de lavage caractérisé en ce que le détergent est un détergent selon l'une quelconque des revendications 1 à 8, le pH est dans la gamme de 7 à 11 et la température est inférieure à 60°C.

14. Procédé de lavage selon la revendication 13, dans lequel la solution de lavage contient le détergent en une quantité dans la gamme de 1 à 5 g/l de solution de lavage.

15. Utilisation d'un détergent selon l'une quelconque des revendications 1 à 8 pour le lavage.


**Revendications pour l'Etat Contractant: AT**

1. Procédé de préparation d'un détergent comprenant un additif enzymatique dont le composant actif est une lipase produite par voie microbiologique, caractérisé en ce que la lipase est une lipase produite à l'aide d'une souche productrice de lipase de *Fusarium oxysporum*, qui produit plus de 10 UL/ml dans les conditions standard de fermentation.

2. Procédé selon la revendication 1, dans lequel la souche productrice de lipase de *Fusarium oxysporum* est DSM 2672.

3. Procédé selon la revendication 1 ou 2, dans lequel l'additif est sous forme de granulès ne formant pas de poussière.

4. Procédé selon la revendication 1 ou 2, dans lequel l'additif est sous forme d'une liquide avec un stabilisant enzymatique.

5. Procédé selon la revendication 3 ou 4, dans lequel l'activité lipasique est dans la gamme de 20 000 à 100 000 UL/g d'additif.

6. Procédé selon l'une quelconque des revendications précédentes, dans lequel l'additif contient en plus de la lipase une enzyme protéolytique.

7. Procédé selon la revendication 6, dans lequel l'activité protéolytique est dans la gamme de 0,5 à 3,0 unités Anson/g d'additif.

8. Procédé selon l'une quelconque des revendications précédentes, dans lequel le détergent contient l'additif enzymatique pour détergent en une quantité de 0,2 à 2,0% p/p.

9. Utilisation d'un additif enzymatique pour détergent dans la préparation d'un détergent selon le procédé de la revendication 3, dans lequel additif le composant actif est une lipase, produite par voie microbiologique, caractérisée en ce que la lipase est une lipase, produite à l'aide d'une souche productrice de lipase de *Fusarium oxysporum* qui produit plus de 10 UL/ml dans les conditions standard de fermentation, sous forme de granulés ne formant pas de poussière.

10. Utilisation d'un additif enzymatique pour détergent dans la préparation d'un détergent selon le procédé de la revendication 4, dans lequel additif le composant actif est une lipase produite par voie microbiologique, caractérisée en ce que la lipase est une lipase, produite à l'aide d'une souche productrice de lipase de *Fusarium oxysporum* qui produit plus de 10 UL/ml dans les conditions standard de fermentation, sous forme d'un liquide avec un stabilisant enzymatique.

11. Utilisation d'une lipase, pouvant être produite à l'aide d'une souche productrice de lipase de *Fusarium oxysporum* qui produit plus de 10 UL/ml dans les conditions standard de fermentation, comme composant actif d'un additif enzymatique pour détergent.

12. Utilisation selon la revendication 11, dans laquelle la souche productrice de lipase de *Fusarium oxysporum* est DSM 2672.

13. Procédé de lavage caractérisé en ce que le détergent est un détergent préparé selon l'une quelconque des revendications 1 à 8, le pH est dans la gamme de 7 à 11 et la température est inférieure à 60°C.

14. Procédé de lavage selon la revendication 13, dans lequel la solution de lavage contient de détergent en une quantité dans la gamme de 1 à 5 g/l de solution de lavage.

15. Utilisation d'un détergent préparé selon l'une quelconque des revendications 1 à 8 pour le lavage.